# EUROPEAN PATENT APPLICATION

(11) **EP 4 799 579 A1**
(43) Date of publication of application: **02.09.2026**
(21) Application number: 25160594.5
(22) Date of filing: 27.02.2025
(51) Int. Cl.: A61B 17/70

(54) **BONE ANCHORING DEVICE**

(71) Applicant: Biedermann Technologies GmbH & Co. KG, 78166 Donaueschingen (DE)
(72) Inventor: BIEDERMANN, Lutz, 78048 VS-Villingen (DE); BIEDERMANN, Timo, 78647 Trossingen (DE); DANNECKER, Berthold, 78112 St. Georgen (DE)
(74) Representative: Prüfer & Partner mbB Patentanwälte · Rechtsanwälte

(57) **Abstract**

A bone anchoring device for coupling a rod to bone is provided, the bone anchoring device including an anchoring element (1) comprising a shank (2) for anchoring in bone and a head (3), a receiving part (5) having a first end (5a) and a second end (5b), a channel (54) for receiving the rod (100), a passage (51) extending from the first end to the second end, the passage (51) defining a central axis (C), and an accommodation space (53) for accommodating the head (3) of the bone anchoring element (1), a pressure element (6) configured to be arranged in the passage (51) and to encompass the head (3) laterally and from a free end of the head (3) to exert pressure onto the head (3) when the head (3) and the pressure element (6) are in the receiving part (5) such that the head (3) is pivotable with respect to the receiving part (5) and can be locked at an angle relative to the receiving part (5); wherein the anchoring element comprises a first cooperating surface (30) and the receiving part comprises a protrusion (57) at an inner wall of the accommodation space (53) providing a second cooperation surface (57a) which cooperates with the first cooperating surface (30) to limit pivoting of the head (3) to a single plane.

## Description

The invention relates to a bone anchoring device comprising a bone anchoring element and a receiving part for coupling a rod to the bone anchoring element. In particular, the bone anchoring device relates to a uniplanar modular bone anchoring device in which the bone anchoring element can pivot relative to a receiving part in a single plane.

Bone anchoring devices comprising a bone anchoring element and receiving part are used in orthopaedic surgery, in particular in spinal surgery, for coupling a rod to the bone anchoring element that is anchored in bone or a vertebra and for connecting several bone anchoring devices by the rod. In a polyaxial bone anchoring device, a head of the bone anchoring element is pivotably received in the receiving part, so that the receiving part can assume various angular positions in multiple planes with respect to the bone anchoring element. In a uniplanar bone anchoring device, the bone anchoring element can pivot in the receiving part only in a single plane. A desired angular position of the bone anchoring element relative to the receiving part in both types, the polyaxial and the uniplanar, can be locked with the aid of a pressure element acting on the head.

Various uniplanar bone anchoring devices are known in the art. For example, a uniplanar bone anchoring device is described in US 7,749,258 B2. The bone anchoring device comprises a bone anchoring element which is pivotable in a receiving part in a single plane around the central axis of the receiving part. Pivoting of the anchoring element prior to locking is limited to the single plane by a form-fit connection between the head of the anchoring element and the pressure element.

US 7,892,259 B2 describes a bone anchoring device comprising a receiving part and a bone anchoring element having a head with a spherically shaped surface portion and a portion recessed from the spherically shaped surface portion, wherein a pin is provided in a bore of the receiving part that engages with the recessed portion of the head such that the anchoring element is pivotable relative to the receiving part around a single axis of rotation.

US 2008/0195159 A1 describes a pedicle screw with a shank and a ball head with two flat parallel surfaces corresponding to respective surfaces of a screw head to determine the mobility thereof, or, respectively, prevent a free rotablity.

It is the object to provide a bone anchoring device and a method of manufacturing the same that has an increased versatility. It is a further object to provide a modular system of such a bone anchoring device and a further receiving part that allows to selectively combine the bone anchoring element with a suitable receiving part depending on the clinical application.

The object is solved by a bone anchoring device of claim 1, by a method of claim 13 and by a system of claim 15. Further developments are given in the dependent claims.

According to an aspect, a bone anchoring device for coupling a rod to bone, includes an anchoring element comprising a shank for anchoring in bone and a head, a receiving part having a first end and a second end, a channel for receiving the rod, a passage extending from the first end to the second end, the passage defining a central axis, and an accommodation space for accommodating the head of the bone anchoring element. The bone anchoring device further comprises a pressure element configured to be arranged in the passage of the receiving part and to encompass the head laterally and from a free end of the head to exert pressure onto the head when the head and the pressure element are in the receiving part such that the head is pivotable with respect to the receiving part and can be locked at an angle relative to the receiving part. The anchoring element comprises first cooperating surface and the receiving part comprises a protrusion at an inner wall of the accommodation space providing a second cooperation surface which cooperates with the first cooperating surface to limit pivoting of the head to a single plane.

The head of the bone anchoring element is insertable in the receiving part from a bottom of the receiving part. Thus, a modular uniplanar bone anchoring device is provided, that permits to select a suitable bone anchoring element and to combine it with the receiving part prior to or during surgery. In particular, the bone anchoring element may be inserted into bone or a vertebra and the receiving part can be mounted on the head of the bone anchoring element *in-situ.*

Moreover, with the receiving part, bone anchoring elements can be used that have an enlarged head which cannot be inserted through the first or top end of the receiving part. Thus, an overall lateral dimension of the receiving part may be kept small. At the same time, a robust bone anchoring device can be provided due to an increased holding force between the head of the bone anchoring element in the pressure element and the receiving part.

In addition, a maximum pivot angle of the bone anchoring element relative to the receiving part may be increased.

According to a further aspect, a modular system comprises a first receiving part with a first pressure element and a second receiving part with second pressure element and the bone anchoring element. The first receiving part comprises the second cooperating surface and the second receiving part lacks the second cooperating surface. Thus, the bone anchoring element can be selectively combined with first receiving part to provide a uniplanar bone choring device or with the second receiving part to provide a poyaxial bone anchoring device.

The receiving part may be manufactured using an additive manufacturing method. This permits to generate the second cooperating surface easily within the receiving part, in particular to generate the protrusion with the second cooperating surface monolithically with an inner wall of the receiving part.

Further features and advantages will become apparent from the description of embodiments by means of the accompanying drawings. In the drawings:
- Fig. 1:: shows a perspective exploded view of a bone anchoring device according to a first embodiment.
- Fig. 2:: shows a perspective view of the bone anchoring device of Fig. 1 in an assembled state.
- Fig. 3:: shows a cross-sectional view of the bone anchoring device of Figs. 1 and 2, the cross-section taken in a plane extending through the central axis and perpendicular to the rod axis of an inserted rod.
- Fig. 4: shows a cross-sectional view of the bone anchoring device of Figs. 1 and 2, the cross-section taken in a plane extending through the central axis and including the rod axis of an inserted rod.

- Fig. 5:: shows a perspective view from a top of a receiving part of the bone anchoring device of Figs. 1 to 4.
- Fig. 6:: shows a perspective view from a bottom of the receiving part of Fig. 5.
- Fig. 7:: shows a top view of the receiving part of Figs. 5 and 6 with a cross-sectional line A-A.
- Fig. 8:: shows a cross-sectional view of the receiving part of Figs. 5 to 7 the cross-section taken along line A-A in Fig. 7.
- Fig. 9:: shows a top view of the receiving part of Figs. 5 and 6 with a cross-sectional line B-B.
- Fig. 10:: shows a cross-sectional view of the receiving part of Figs. 5 to 9 the cross-section taken along line B-B in Fig. 9.
- Fig. 11:: shows a perspective view from a top of a pressure element of the bone anchoring device of Figs 1 to 4.
- Fig. 12:: shows a perspective view from a bottom of the pressure element of Fig. 11.
- Fig. 13:: shows a top view of the pressure element of Figs. 11 and 12 with a cross-sectional line D-D.
- Fig. 14:: shows a cross-sectional view of the pressure element of Figs. 11 to 13, the cross-section taken along line D-D in Fig. 13.
- Figs. 15 to 18:: show steps of assembling the bone anchoring device of Figs. 1 to 4.
- Fig. 19:: shows a cross-sectional view of a further embodiment of the bone anchoring device, the cross-section taken in a plane extending through the central axis and perpendicular to the longitudinal axis of a rod support surface.
- Fig. 20:: shows a cross-sectional view of the further embodiment of the bone anchoring device of Fig. 19, the cross-section taken in a plane extending through the central axis and including the longitudinal axis of the rod support surface.
- Fig. 21:: shows a cross-sectional view of a still further embodiment of the bone anchoring device, the cross-section taken in a plane extending through the central axis and perpendicular to the longitudinal axis of the rod support surface.
- Fig. 22:: shows a perspective view of a modular system of a bone anchoring device with a first receiving part with a first pressure element according to Figs. 1 to 21 and a second receiving part with a second pressure element.
- Fig. 23:: shows a cross-sectional view of the system of Fig. 22.

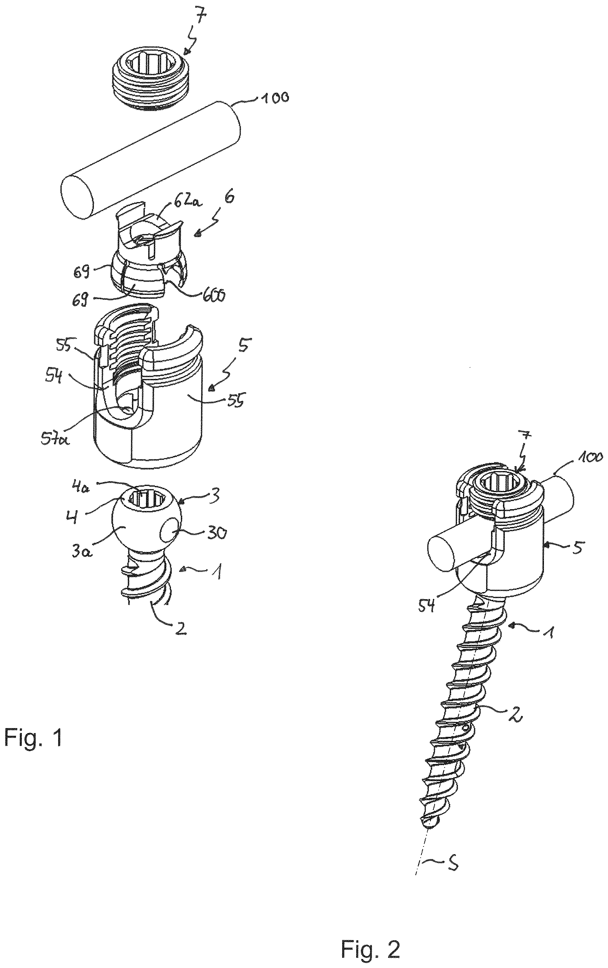

Referring to Figs. 1 to 4, the bone anchoring device includes a bone anchoring element 1 having a shank 2 for anchoring in bone or in a vertebra and a head 3. The shank 2 has a bone engagement structure, such as a bone thread, and defines a screw axis S. The head 3 is shaped as a segment of a sphere, thus comprises a spherically-shaped outer surface portion 3a that may include a region with a greatest outer diameter E of the head 3. In addition, the head 3 comprises at its free end 4 an engagement portion 4a for engagement with a tool, such as a driver. Two opposite planar surface portions 30 are provided at opposite sides of the head 3. The planar surface portions 30 may have a circular shape. A size of the planar surface portions 30 may be such that between each planar surface portion 30 and the free end of the head 3 comprising the engagement portion 4 and between each planar surface portion 30 and the shank, there is still a spherically-shaped outer surface portion 3a. In other words, the planar surface portions 30 are recessed from the spherically-shaped outer surface 3a. Moreover, the greatest outer diameter E of the head extends preferably through a center of the planar surface portions 30 in an axial direction.

Further, the bone anchoring device includes a receiving part for receiving the head 3 of the bone anchoring element 1 and for receiving a rod 100 which is configured to connect at least two or more bone anchoring devices. In the receiving part 5, a pressure element 6 is provided for exerting pressure onto the head 3 of the bone anchoring element 1 and for providing support for the rod 100. To lock the head 3 and the rod 100 in the receiving part 5, a locking element 7 in the form of an inner screw or set-screw may be provided.

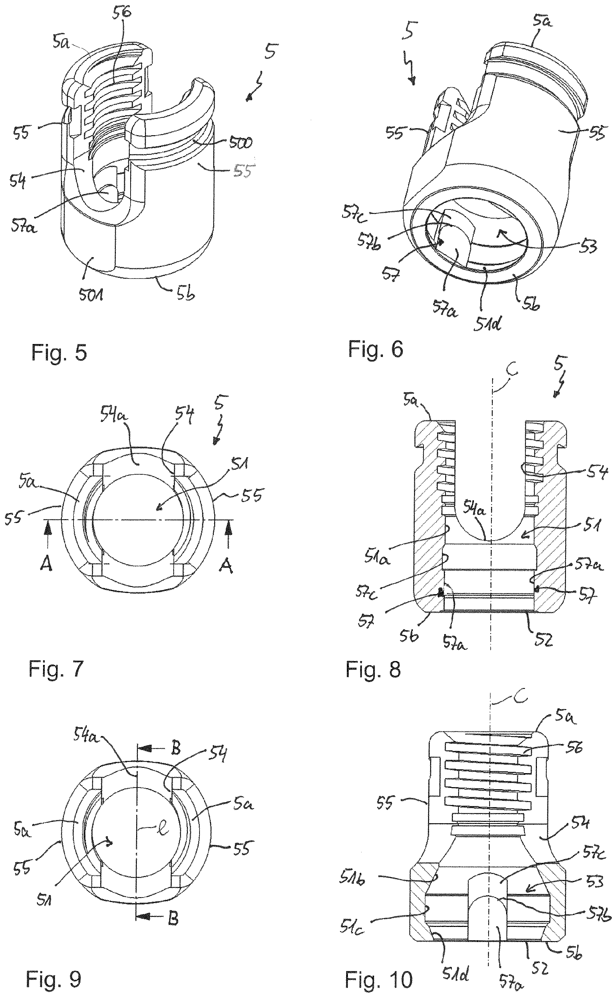

Referring in addition to Figs. 5 to 10, the receiving part 5 will be explained in greater detail. The receiving part 5 has a first or top end 5a and a second or bottom end 5b opposite to the top end 5a. In general, the receiving part 5 may have a substantially cylindrical outer shape with a central longitudinal axis C extending through the top end 5a and the bottom end 5b. Coaxially to the central axis C, a passage 51 is provided that extends from the top end 5a to the bottom end 5b and that forms an opening 52 at the bottom end 5b. At a distance from the top end 5a, the passage 51 widens into an accommodation space 53 that is configured to receive the head 3 and at least a portion of the pressure element 6 therein. The receiving part 5 further has a substantially U-shaped recess 54 starting at the top end 5a and extending in a direction of the bottom end 5b. By means of the U-shaped recess 54, two free legs 55 are formed and define a channel that is open towards the first end 5a for receiving the rod 100. On an inner surface of the legs 55, an internal thread 56 is formed, which is in the exemplary embodiment a square thread or another flat thread.

As can be seen in particular in Figs. 6, 8 and 10, a portion of the passage 51 starting from the top end 5a is formed by a substantially cylindrical bore 51a with an inner diameter that is preferably smaller than a greatest outer diameter E of the head 3. The bore 51a may extend below the bottom 54a of the U-shaped recess 54 in an axial direction. Following the bore 51a, the passage widens in a widening portion 51b into a substantially cylindrical portion 51c that has an inner diameter greater than the greatest outer diameter E of the head 3 and greater than an outer diameter of the pressure element 6 with the inserted head 3. Following the cylindrical section 51c, the passage 51 narrows towards the bottom end 5b in a narrowing portion 51d, for example a conically narrowing portion. Since the maximum width of the opening 52 at the bottom end 5b is greater than the greatest outer diameter of the head 3, the head 3 is insertable into the receiving part 5 from the bottom end 5b. If the largest inner width of the bore 51a is smaller than the greatest outer diameter E of the head 3, the bone anchoring element 1 can only be inserted into the receiving part through the opening 52 at the bottom end 5b.

At a circumferential position corresponding to the center of each leg 55 and substantially adjacent to the lower opening 52, a protrusion 57 is formed that comprises a planar surface portion 57a extending in an axial direction substantially parallel to the central axis C. Hence, two opposite protrusions 57 with planar surface portions 57a are formed. The planar surface portion 57a may comprise a circular upper end 57b and substantially straight side edges. The area provided by the planar surface portions 57a of the protrusions may substantially cover the area of the planar surface portions 30 provided at the head 3. In the axial direction, the planar surface portion 57a may extend into the middle portion 51c of the accommodation space 53. Each protrusion 57 is surrounded in the axial direction towards the top end 5a by a base portion 57c with a reduced thickness that extends axially into the widening portion 51b of the passage 51.

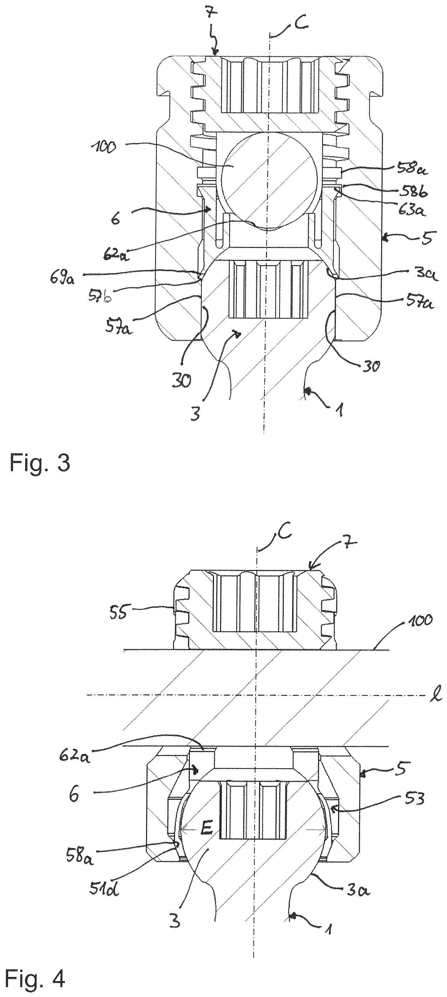

As can be seen in particular in Fig. 3, the distance between the planar surface portions 57a is such that, when the head 3 is inserted through the lower opening 52 into the receiving part 5 in an orientation where the planar surface portions 57a of the receiving part and the corresponding planar surface portions 30 of the head 3 face each other, each planar surface portion 57a engages the corresponding planar surface portions 30 of the head 3. Hence, the planar surface portions 30 at the head 3 form each a first cooperating surface and the planar surface portions 57a of the receiving part form each a second cooperating surface configured to cooperate with a corresponding first cooperating surface at the head 3. It shall be noted that the protrusions 57 are monolithic with the inner wall of the accommodation space of the receiving part 5.

A circumferential first groove 58a may be provided in the inner wall of the legs 55 at a distance from the bottom 54a of the U-shaped recess 54. The first groove 58a may provide a stop for restricting an upward movement of the pressure element 6 towards the top end 5a when the pressure element 6 is assembled with the receiving part 5 and when the pressure element 6 is in an insertion position. Between the circumferential first groove 58a and the bottom of the U-shaped recess 54a in the axial direction, there may be a circumferential second groove 58b in the inner wall for engagement with a portion of the pressure element 6 to secure a pre-locking position of the pressure element 6.

Moreover, in the outer surface of each of the legs 55 at a distance from the top end 5a, a circumferential tool engagement groove 500 may be formed for engagement with a tool or an instrument. At an outer surface of the receiving part 5 aligned with the U-shaped recess, two opposite planar areas 501 may be provided that reduce an overall width of the receiving part.

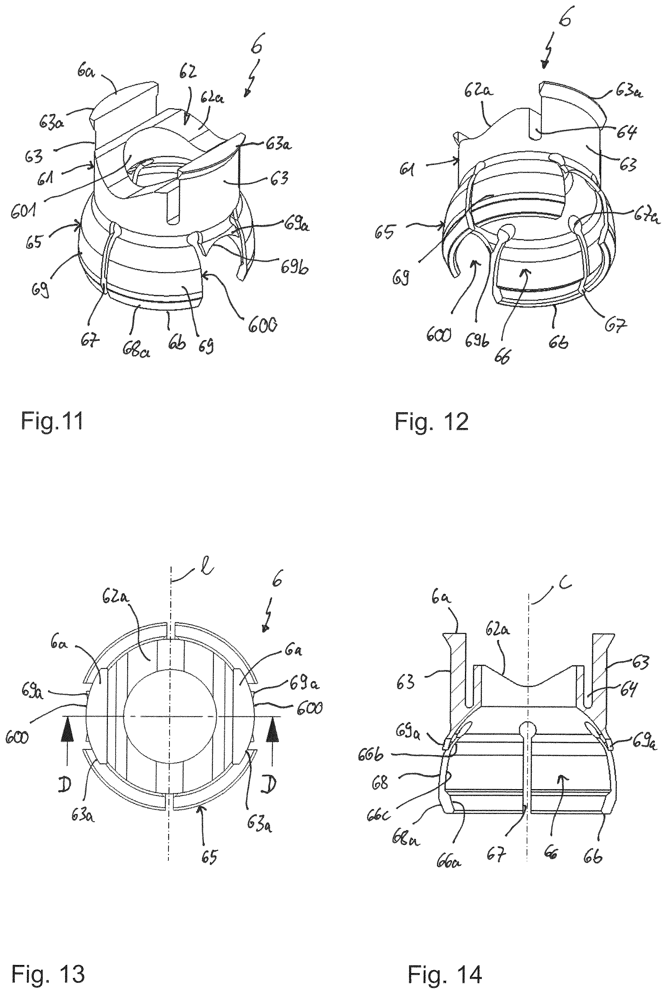

Referring further to Figs. 11 to 14, the pressure element 6 will be described. Preferably, the pressure element 6 is a monolithic piece which is configured to be arranged in the passage 51. The pressure element 6 is further configured to encompass the head 3 laterally and from a free end of the head 3 to exert pressure onto the head 3 when the head 3 and the pressure element 6 are in the receiving part such that the head 3 can be locked such that the shank axis S forms a desired angle with the central axis C of the receiving part. In greater detail, the pressure element 6 has a first or top end 6a and a second or bottom end 6b. Adjacent to the top end, the receiving part 6 comprises a first portion 61 with a substantially cylindrical outer surface with an outer diameter that allows the pressure element 6 to move axially within the bore 51a of the passage 51 of the receiving part 5. At the top end 6a, a rod receiving recess 62 is formed that comprises a rod support surface 62a. The rod support surface 62a may have a substantially V-shaped cross-section with a longitudinal axis l extending substantially perpendicular to a cylinder axis of the first portion 61 that coincides with the central axis C of the receiving part 5 when the pressure element 6 is arranged in the receiving part 5. The depth of the rod receiving recess 62 may be smaller than a diameter of the rod 100. Hence, when the rod 100 rests on the rod support surface 62a, the rod 100 projects over the top end 6a of the pressure element 6 as shown, for example, in Fig. 3. The V-shape of the rod support surface 62a enables use of rods with different diameters.

The rod receiving recess 62 is shaped such that two free upstanding legs 63 are formed that may be spaced apart from the rod support surface 62a on each side by a groove 64. By means of this, the legs 63 are slightly flexible in a direction transverse to the longitudinal axis l of the rod support surface 62a. A free end of each of the legs 63 may have a radially outwardly protruding rim 63a, and upper surface of which forms the top end 6a of the pressure element 6. To secure an insertion position of the pressure element 6, the radially outwardly protruding rim 63a is configured to engage the first groove 58a provided at the inner surface of the legs 55 of the receiving part 5. To secure a pre-locking position of the pressure element 6 in the receiving part 5, the radially outwardly protruding rim 63a is configured to engage the second groove 58b provided at the inner surface of the legs 55.

A second portion 65 of the pressure element 6 has a substantially cap-like shape that is configured to receive the head 3 of the bone anchoring element 1. The second portion 65 of the pressure element 6 comprises a hollow head receiving portion 66 with an opening at the bottom end 6b for inserting the head 3. The head receiving portion 66 may have a lower and an upper substantially spherical section 66a, 66b that are shaped so as to mate with the spherical outer surface portion 3a of the head 3. An intermediate section 66c may have a greater diameter for facilitating the insertion of the head 3. In addition, a plurality of axials slits 67 are formed that are open to the second end 6b and that may have an enlarged, preferably rounded, end portion 67a. The slits 67 extend along an axial length that covers at least the intermediate section 66c which has the greatest inner diameter. In general, the number, shape and size of the slits are selected such that a desired flexibility is achieved that allows the expansion of the head receiving portion 66 when the head 3 is inserted through the bottom end 6b and the compression around an inserted head 3. The size of the head receiving portion 66 may also be such that the head 3 can be held therein by friction prior to final locking of the head 3 in the receiving part 5.

An outer surface 68 of the head receiving portion 66 may be rounded and may have a greater outer diameter than the outer diameter of the cylindrical first section 61. Adjacent to the bottom end 6b, the outer surface 68 comprises a narrowing portion 68a, for example, a conically narrowing portion that is configured to engage the narrowing portion 51d at the bottom region of the accommodation space 53 of the receiving part 5.

By means of the slits 67 the wall of the head receiving portion 66 is divided in circumferentially spaced-apart wall portions 69. It shall be noted that two opposite slits are aligned with the longitudinal axis l of the rod receiving recess 62 and four slits are arranged in pairs symmetrically to the center of the legs 63 in the circumferential direction, thereby forming opposite wall portions. These wall portions are partially removed such that a cutout 600 is formed between the slits framing the wall portions 69a. The remainder 69a of the wall portions may have a concavely curved lower edge 69b that mates the convexly curved upper edge 57b of the protrusion 57 which has the planar surface portion 57a of the receiving part 5. When the pressure element 6 is in the receiving part 5 such that the rod receiving recess 62 is aligned with the U-shaped recess 54 of the receiving part 5, the protrusions 57 with the planar surface portions 57a are configured to extend through the cutouts 600 into the hollow head receiving portion 66 to contact the planar surface portions 30 of the head 3. The circumferential width of the cutout 600 may be smaller than the circumferential width of one of the wall portions 69 as can be seen in Fig. 11.

In addition, the pressure element 6 comprises a coaxial opening or bore 601 that allows access to the tool engagement recess 4a of an inserted head 3 with a tool, such as a driver.

The parts and portions of the bone anchoring device may be made of any material, preferably, however, of titanium or stainless steel, or a bio-compatible metal or metal alloy, or plastic material. For bio-compatible alloys, a NiTi alloy, for example Nitinol, may be used. Other materials that can be used are, for example, magnesium or magnesium alloys, bio-compatible plastic materials, for example, polyetheretherketone (PEEK) or poly-L-lactide acid (PLLA). The parts can be made of the same or of different materials from one another.

A method of manufacturing the bone anchoring device includes manufacturing at least the receiving part 5 with an additive manufacturing method, more specifically, an additive layer manufacturing method. In such a method, the receiving part is built-up by layer-wise deposition of a building material and solidifying or melting the building material in each layer at positions corresponding to the cross-section of the receiving part and the respective layer. A suitable method is, for example, selective laser sintering (SLS) or selective laser melting (SLM) in which the building material is a powder, such as a metal powder or a plastic powder, and a laser is used to melt the powder. Consecutive layers of the building material are applied to a support surface or a previous layer, and the laser beam is steered to the positions of a layer which correspond to the cross-section of the receiving part in each layer. Thus, the receiving part is built-up in an additive manner. The building material may preferably be a titanium powder or a stainless steel powder or a suitable metal alloy. Alternatively, an electron beam may be used to melt the building material. Also, other known methods of powder-based three-dimensional printing in which layers of a powder material are deposited and solidified by applying a binder material at positions corresponding to the receiving part may be used. Still further additive manufacturing methods, for example, fuse deposition modelling (FDM) may also be used.

With an additive manufacturing method, the protrusions 57 in the receiving part may be easily formed based on a three-dimensional computer aided design (CAD) model of the receiving part to be manufactured.

It shall be noted that the additive manufacturing method, in particular an additive layer manufacturing method, influences the outer appearance of the receiving part manufactured by such a method. For example, the layers may be visible on the surface of the finished part, even it is post-treated, such as polished, edged, coated or otherwise treated. It may also be possible to identify the traces of the laser or electron beam when inspecting the manufactured part. Hence, the use of an additive manufacturing method, in particular an additive layer manufacturing method, can be distinguished based on the finished part compared to a conventional subtractive manufacturing method.

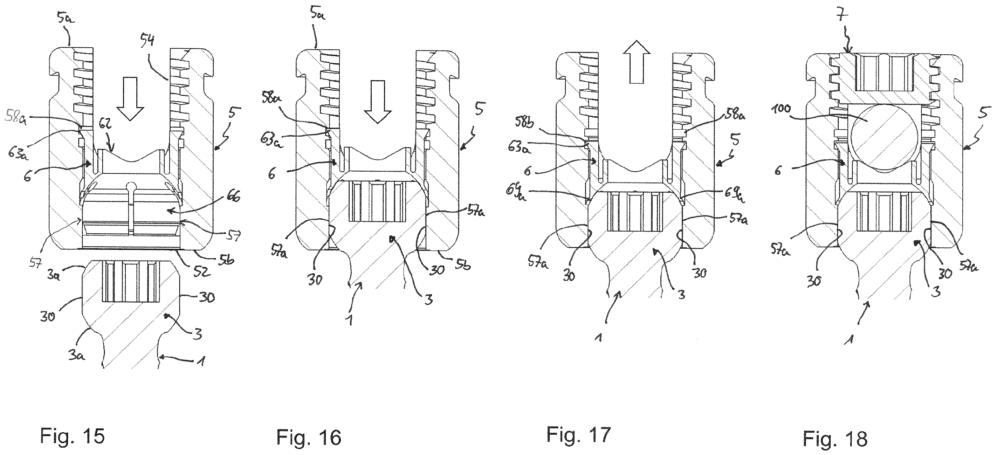

Referring to Figs. 15 to 18, steps of assembling a bone anchoring device will be described.

As depicted in Fig. 15, the pressure element 6 and the receiving part 5 are pre-assembled such that the rod receiving recess 62 of the pressure element 6 and the U-shaped recess 54 of the receiving part 5 are aligned and the pressure element 6 is in an insertion position. In the insertion position, the outer rim 63a of the pressure element 6 engages the upper wall of the first groove 58a, so that the pressure element 6 is prevented from moving toward the top end 5a of the receiving part 5 when the head 3 is inserted into the head receiving portion 66 of the pressure element 6. One possibility to pre-assemble the pressure element 6 with the receiving part 5 is to insert the pressure element 6 from the top end 5a of the receiving part 5 by slightly compressing the head receiving portion 66 and the legs 63 of the pressure element.

In the insertion position, the head receiving portion 66 of the pressure element 6 is located in the widened section 51b and 51c of the accommodation space 53. The cutouts 600 are circumferentially aligned with the protrusions 57 of the receiving part 5.

Still referring to Fig. 15, the receiving part 5 with the pressure element 6 is oriented relative to the bone anchoring element 1 such that the planar outer surface portions 30 of the head 3 are aligned with the protrusions 57 when the head 3 is inserted. The head 3 enters through the lower opening into the accommodation space 53 of the receiving part 5 and into the head receiving portion 66 of the pressure element 6.

Next, as shown in Fig. 16, the receiving part 5 with pressure element 6 is mounted on the head 3.

As depicted in Fig. 17, by pulling the receiving part 5 relative to the bone anchoring element 1, the outer rim 63a engages the second groove 58b. Since the legs 63 of the pressure element 6 are slightly flexible, the outer rim 63a can disengage from the first groove 58a and can engage the second groove 58b. The pressure element 6 assumes a pre-locking position, in which the narrowing outer surface portion 68a of the pressure element 6 engages the narrowing inner surface portion 51d of the receiving part 5. As a result, in the pre-locking position, the lower opening 52 is reduced by the pressure element 6 so that the head 3 is prevented from being removed through the lower opening 52. The head 3 can pivot only in a single plane that includes the longitudinal axis of the rod support surface and the central axis.

Fig. 18 shows the bone anchoring device with the inserted rod 100 and the locking element 7. Once a suitable angular position of the receiving part 5 relative to the bone anchoring element 1 has been established, the rod 100 is inserted and the locking element 8 is inserted and tightened to lock the head 3 and the rod 100 in the receiving part 5.

In the clinical use, the bone anchoring device can be used in a first manner in which the bone anchoring element 1 is pre-assembled with the coupling device comprising the receiving part 5 and the pressure element 6 outside a patient's body or in a second manner in which the bone anchoring element 1 is already inserted in bone or in a vertebra and the receiving part with the pressure element is mounted *in-situ* on the head 3 of the bone anchoring element 1. In the case of an *in-situ* mounting, the planar surfaces 30 of the head 3 must be oriented properly with respect to the planar surfaces 57a of the receiving part 5. This may be achieved, for example, with the aid of an instrument (not shown) that is configured to indicate the position of the planar surfaces.

Usually, a plurality of polyaxial bone anchoring devices are inserted into bone parts or into vertebrae, in particular into the pedicles of vertebrae. The receiving parts 5 are then aligned so that the rod 100 can be received in the U-shaped recesses 54 of two or more of the bone anchoring devices.

Due to the design of the receiving part and the pressure element, a bone anchoring element 1 can be used with a head 3 having a large outer diameter. For example, instead of a head with a greatest outer diameter of 7 mm, a bone anchoring element with a head of a greatest outer diameter of 8 mm can be used. Moreover, the bone anchoring element 1 can pivot in the receiving part to a greater maximum angle as compared to bone anchoring devices with smaller heads. The maximum pivot angle that the shank axis S can form with the central axis C may be 35° or more. Due to the large head, a stable and robust implant with increased holding force can be provided.

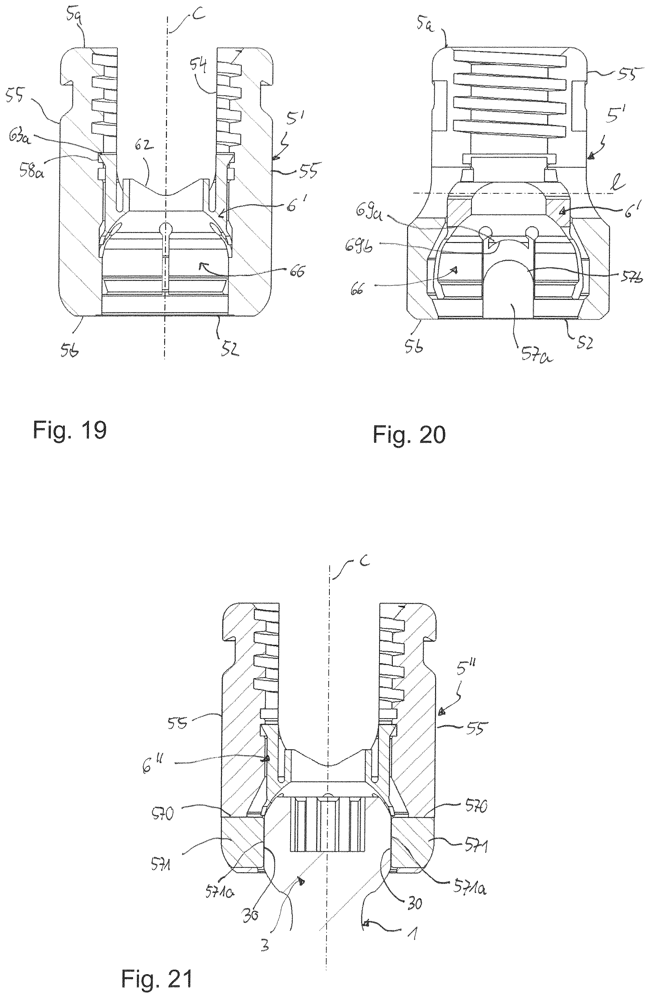

Fig. 19 and 20 show a further embodiment of the receiving part with pressure element of the bone anchoring device. Parts and portions that are identical or highly similar to the previous embodiment are marked with the same reference numerals and the description thereof will not be repeated. The receiving part 5' and the pressure element 6' in this embodiment are manufactured as one single part with an additive manufacturing method. Hence, the three-dimensional CAD-model is the model of a combined part comprising the receiving part 5' and the pressure element 6'. The cross-sectional data of the combined part in each layer are used to control the solidifying unit, such as a laser, in the respective layer. Laser sintering or laser melting, or electron beam melting may be used. In such a method, however, the adding of support structures may become necessary to allow manufacturing of complex shapes including overhangs. The support structures may be removed afterwards.

In addition, the additive lithography-based metal manufacturing (LMM) technique may be used which allows the manufacturing without using support structures. A raw material is used that consists of metal particles which are dispersed in an organic binder phase. At room temperature, the raw material is a solid block. By the application of heat, for example, by a heated blade, thin layers of the raw material can be deposited on a building platform. Each layer of raw material is then selectively exposed to light to trigger photopolymerization of the organic binder phase. The layer-wise application and photopolymerization process is repeated until the entire part is manufactured layer by layer. The surrounding non-cured raw material may be solidified by active cooling. Hence, the printed part is finally incorporated in a block of raw material as a green part. The unused raw material supports the green part such that no additional support structures are required. The green part can then be released by liquifying the raw material block and removing the organic binder. Thereafter, the green part is sintered in a sintering oven to produce the final part.

Fig. 21 shows a still further embodiment which differs from the previous embodiment by the design of the receiving part. The receiving part 5" lacks the protrusion 57 with the planar surface portion 57a which is monolithic with the wall of the accommodation space 53. Instead, the receiving part 5" comprises adjacent to the bottom end 5b and circumferentially at the position of the middle of the legs 55 two opposite transverse through holes 570 that are sized and shaped to receive pins 571, respectively, for example in a press-fit manner. The pins 571 comprise each a planar front surface 571a that is configured to cooperate with the planar surface portions 30 of the head 3. An axial length of the pins 571 may be such that the front surface 571a abuts against the planar surface portion 30 of the head 3 to limit a pivoting motion of the head 3 to a single plane.

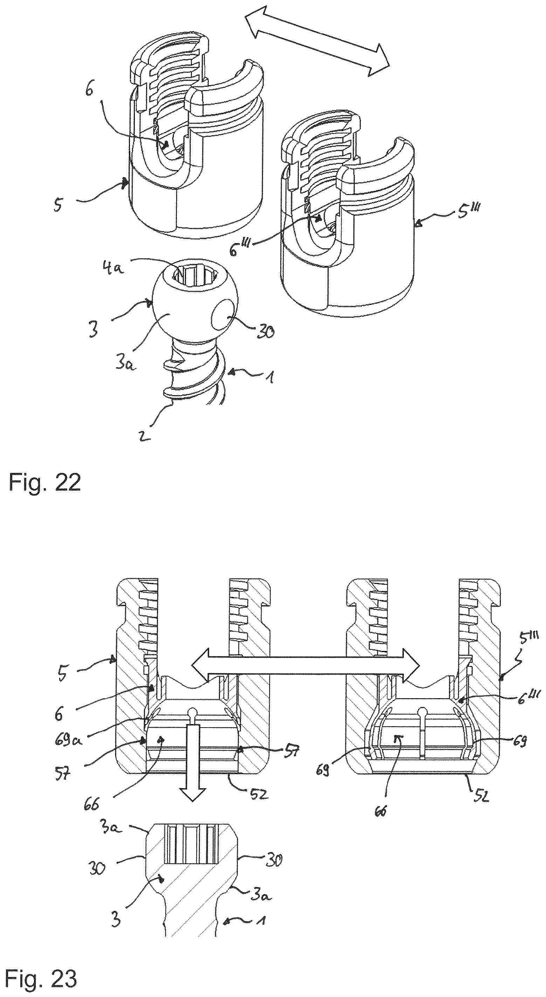

Referring to Figs. 22 and 23, a modular system of a bone anchoring element 1 and a first receiving part 5 with first pressure member 6 and a second receiving part 5‴ with a second pressure member 6‴ is shown. The bone anchoring element 1 is identical to the bone anchoring element 1 of the previous embodiments, and the first receiving part 5 with the first pressure member 6 is identical to the receiving part 5, 5', 5" with pressure member 6, 6', 6" according to Figs. 1 to 21.

The second receiving part 5‴ with the second pressure member 6‴ is configured to form together with the bone anchoring element 1 a polyaxial bone anchoring device. For this purpose, the second receiving part 5‴ lacks the protrusion 57. Instead, the widening sections 51b, 51c and the narrowing section 51b of the accommodation space 53 are rotationally symmetrical. As a result, the receiving part 5‴ is free from a second cooperating surface that limits pivoting of the head to a single plane. The second pressure member 6‴ lacks the cutouts 600. Instead, two opposed full wall portions 69 are present.

Thus, the head can pivot in the receiving part 5‴ with the pressure member 6‴ in multiple planes. Depending on the clinical requirement, a user can select from the modular system either a uniplanar bone anchoring device by combining the bone anchoring element 1 with the first receiving part 5, 5', 5" and the first pressure element 6, 6', 6" or by combining the bone anchoring element 1 with the second receiving part 5‴ with the second pressure element 6"'. This gives a surgeon a considerable choice of implants.

Modification of the bone anchoring device of the first embodiments may be conceivable. For example, the protrusions 57 with the planar surface portions 57a may be located circumferentially at another position, for example, they may be aligned with the U-shaped recess 54 of the receiving part 5. The single pivot plane in this case is perpendicular to the rod axis or is, in other words, perpendicular to the longitudinal axis of the U-shaped recess 54 or the rod receiving recess 62 of the pressure element 6.

The cooperating surfaces may have another shape, in particular, they need not to be planar. For example, only one surface can be planar and the other can be convex. Other shapes may be conceivable.

In a further modification, the system may also comprise a second bone anchoring element (not shown) with additional opposite planar surface portions that are spaced apart from the planar surface portions 30 by 90° and a third receiving part with a third pressure member (also not shown) that comprise additional protrusions 57 in the receiving part that are spaced apart by 90° in the circumferential direction. The corresponding pressure member (not shown) may comprise additional cutouts that are spaced apart from the cutouts by 90° in the circumferential direction. When the second bone anchoring element is combined with the third receiving part and the third pressure element, a monoaxial bone anchoring device can be provided in which the bone anchoring element is prohibited from pivoting in the receiving part.

Instead of the locking member 7 being a set screw all other kinds of locking assemblies known in the art may be used. For the bone anchoring element all types of bone anchoring elements that are suitable for anchoring in bone or a vertebra, such as bone screws, bone nails, etc. may be used. As used in the present specification and the appended claims, the term "rod" shall be understood as including any elongate member, regardless of the cross-sectional shape of the elongate member. Specifically, a spinal stabilization rod as used herein may have a substantially circular, oval, or angular cross-section. Such a cross-section may further vary along a length of the rod. The rod may be stiff or flexible.

Moreover, the accommodation space of the receiving part and the pressure element may have a design that allows to pivot the bone anchoring element to a greater pivot angle to one side compared to other sides.

The shape of the parts is not limited to the detailed shape as shown in the figures. Deviations may be possible and encompassed by the disclosure.

## Claims

1. A bone anchoring device for coupling a rod to bone, including
an anchoring element (1) comprising a shank (2) for anchoring in bone and a head (3),
a receiving part (5) having a first end (5a) and a second end (5b), a channel (54) for receiving the rod (100), a passage (51) extending from the first end to the second end, the passage (51) defining a central axis (C), and an accommodation space (53) for accommodating the head (3) of the bone anchoring element (1),
a pressure element (6) configured to be arranged in the passage (51) and to encompass the head (3) laterally and from a free end (4) of the head (3) to exert pressure onto the head (3) when the head (3) and the pressure element (6) are in the receiving part (5) such that the head (3) is pivotable with respect to the receiving part (5) and can be locked at an angle relative to the receiving part (5);
wherein the anchoring element comprises a first cooperating surface (30) and the receiving part comprises a protrusion (57) at an inner wall of the accommodation space (53) providing a second cooperation surface (57a) which cooperates with the first cooperating surface (30) to limit pivoting of the head (3) to a single plane.

2. The bone anchoring device of claim 1, wherein the head (3) comprises a spherically-shaped outer surface portion (3a) and the first cooperating surface (30) is a portion recessed from the spherically-shaped surface portion (3a).

3. The bone anchoring device of claim 1 or 2, wherein the second cooperating surface (57a) is monolithic with an inner wall of the accommodation space (53).

4. The bone anchoring device of one of claims 1 to 3, wherein the first cooperating surface (30) and the second cooperating surface (57a) each comprise a planar portion and wherein the planar portions cooperate to limit pivoting to a single plane.

5. The bone anchoring device of claim 1 or 2, wherein two first cooperating surfaces (30) and two second cooperating surfaces (57a) are provided that are opposite to each other, respectively.

6. The bone anchoring device of one of claims 1 to 5, wherein the passage (51) comprises an opening (52) at the second end (5b) that has a width so as to allow insertion of the head (3) through the opening (52) only in an orientation in which the first cooperating surface (30) and the second cooperating surface face (57a) are facing each other.

7. The bone anchoring device of one of claims 1 to 6, wherein the second cooperating surface (57a) is provided adjacent to the opening (52).

8. The bone anchoring device of one of claims 1 to 7, wherein the pressure element (6) comprises a head receiving portion (66) that extends from an upper portion of an inserted head (3) towards the shank (2) beyond a portion with the greatest outer width (E)of the head (3) and wherein at least one opening (600) is provided in the head receiving portion (66) that allows the first cooperating surface (30) to contact the second cooperating surface when the head (3) is in the head receiving portion (66).

9. The bone anchoring device of claim 8, wherein a wall defining the head receiving portion (66) comprises slits (67) rendering the wall flexible and wherein the opening (600) is provided by a cutout between two adjacent slits (67).

10. The bone anchoring device of claim 8 or 9, wherein the pressure element (6) comprises a rod support surface (62a) defining an orientation of the rod axis and wherein the at least one opening (600) is oriented transverse to the rod axis.

11. The bone anchoring device of one of claims 8 to 10, wherein the protrusion (57) is configured to extend through the opening (600).

12. The coupling device of one of claims 8 to 11, wherein the receiving part (5) has near the opening (52) a narrowing portion (51d) configured to cooperate with a corresponding narrowing portion (68a) of the pressure element (6) to clamp the head (3).

13. A method for manufacturing a bone anchoring device of one of claims 1 to 12, the method comprising manufacturing by an additive manufacturing method at least the receiving part (5) with the steps
providing computer data of a three-dimensional (3D) model of the receiving part (5),
manufacturing the receiving part (5) based on the computer data layer by layer from a building material according to a cross-section of the portion of the receiving part (3) in the corresponding layer;
preferably wherein the additive manufacturing method comprises one of laser sintering (SLS), laser melting (SLM) or electron beam melting (EBM) or lithography-based metal manufacturing (LMM).

14. The method of claim 13, wherein the receiving part (5) and the pressure element (6) are manufactured together such that the pressure element (6) is within the receiving part (5), preferably without using support structures.

15. A system of a bone anchoring device of one of claims 1 to 12 wherein the receiving part (5) is a first receiving part and the pressure element (6) is a first pressure element and wherein a second receiving part (5‴) with a second pressure element (6"') is provided, wherein the second receiving part (5‴) is free from a second cooperating surface (57) such that the bone anchoring element (1) is configured to pivot in multiple planes.

## Amended claims

### Amended claims in accordance with Rule 137(2) EPC.

1. A bone anchoring device for coupling a rod to bone, including
an anchoring element (1) comprising a shank (2) for anchoring in bone and a head (3),
a receiving part (5) having a first end (5a) and a second end (5b), a channel (54) for receiving the rod (100), a passage (51) extending from the first end to the second end, the passage (51) defining a central axis (C), and an accommodation space (53) for accommodating the head (3) of the bone anchoring element (1),
a pressure element (6) configured to be arranged in the passage (51) and to encompass the head (3) laterally and from a free end (4) of the head (3) to exert pressure onto the head (3) when the head (3) and the pressure element (6) are in the receiving part (5) such that the head (3) is pivotable with respect to the receiving part (5) and can be locked at an angle relative to the receiving part (5);
wherein the anchoring element comprises a first cooperating surface (30) and the receiving part comprises a protrusion (57) at an inner wall of the accommodation space (53) providing a second cooperation surface (57a) which cooperates with the first cooperating surface (30) to limit pivoting of the head (3) to a single plane,
wherein the pressure element (6) comprises a head receiving portion (66) that extends from an upper portion of an inserted head (3) towards the shank (2) beyond a portion with the greatest outer width (E)of the head (3) and wherein at least one opening (600) is provided in the head receiving portion (66) that allows the first cooperating surface (30) to contact the second cooperating surface when the head (3) is in the head receiving portion (66) .

2. The bone anchoring device of claim 1, wherein the head (3) comprises a spherically-shaped outer surface portion (3a) and the first cooperating surface (30) is a portion recessed from the spherically-shaped surface portion (3a).

3. The bone anchoring device of claim 1 or 2, wherein the second cooperating surface (57a) is monolithic with an inner wall of the accommodation space (53).

4. The bone anchoring device of one of claims 1 to 3, wherein the first cooperating surface (30) and the second cooperating surface (57a) each comprise a planar portion and wherein the planar portions cooperate to limit pivoting to a single plane.

5. The bone anchoring device of claim 1 or 2, wherein two first cooperating surfaces (30) and two second cooperating surfaces (57a) are provided that are opposite to each other, respectively.

6. The bone anchoring device of one of claims 1 to 5, wherein the passage (51) comprises an opening (52) at the second end (5b) that has a width so as to allow insertion of the head (3) through the opening (52) only in an orientation in which the first cooperating surface (30) and the second cooperating surface face (57a) are facing each other.

7. The bone anchoring device of one of claims 1 to 6, wherein the second cooperating surface (57a) is provided adjacent to the opening (52).

8. The bone anchoring device of one of claims 1 to 7, wherein a wall defining the head receiving portion (66) comprises slits (67) rendering the wall flexible and wherein the opening (600) is provided by a cutout between two adjacent slits (67).

9. The bone anchoring device of one of claims 1 to 8, wherein the pressure element (6) comprises a rod support surface (62a) defining an orientation of the rod axis and wherein the at least one opening (600) is oriented transverse to the rod axis.

10. The bone anchoring device of one of claims 1 to 9, wherein the protrusion (57) is configured to extend through the opening (600).

11. The bone anchoring device of one of claims 1 to 10, wherein the receiving part (5) has near the opening (52) a narrowing portion (51d) configured to cooperate with a corresponding narrowing portion (68a) of the pressure element (6) to clamp the head (3).

12. A method for manufacturing a bone anchoring device of one of claims 1 to 11, the method comprising manufacturing by an additive manufacturing method at least the receiving part (5) with the steps
providing computer data of a three-dimensional (3D) model of the receiving part (5), manufacturing the receiving part (5) based on the computer data layer by layer from a building material according to a cross-section of the portion of the receiving part (5) in the corresponding layer.

13. The method of claim 12, wherein the additive manufacturing method comprises one of laser sintering (SLS), laser melting (SLM) or electron beam melting (EBM) or lithography-based metal manufacturing (LMM).

14. The method of claim 12 or 13, wherein the receiving part (5) and the pressure element (6) are manufactured together such that the pressure element (6) is within the receiving part (5), preferably without using support structures.

15. A system of a bone anchoring device of one of claims 1 to 11 wherein the receiving part (5) is a first receiving part and the pressure element (6) is a first pressure element and wherein a second receiving part (5‴) with a second pressure element (6‴) is provided, wherein the second receiving part (5‴) is free from a second cooperating surface (57) such that the bone anchoring element (1) is configured to pivot in multiple planes.
